(19) Europäisches Patentamt
European Patent Office
Office européen des brevets

(11) **EP 3 773 791 B1**

(12) **EUROPÄISCHE PATENTSCHRIFT**

(45) Veröffentlichungstag und Bekanntmachung des Hinweises auf die Patenterteilung:
**12.02.2025 Patentblatt 2025/07**

(21) Anmeldenummer: **19717457.6**

(22) Anmeldetag: **08.04.2019**

(51) Internationale Patentklassifikation (IPC):
*A61M 1/30* (2006.01)     *A61M 1/34* (2006.01)
*A61M 1/36* (2006.01)

(52) Gemeinsame Patentklassifikation (CPC):
**A61M 1/30; A61M 1/305; A61M 1/3413;
A61M 1/3427; A61M 1/3434; A61M 1/3622;
A61M 1/36224; A61M 1/36225;** A61M 1/362262;
A61M 1/362265; A61M 2205/12

(86) Internationale Anmeldenummer:
**PCT/EP2019/058762**

(87) Internationale Veröffentlichungsnummer:
**WO 2019/197314 (17.10.2019 Gazette 2019/42)**

(54) **STEUER- ODER REGELVORRICHTUNG UND BLUTBEHANDLUNGSVORRICHTUNG FÜR DIE SINGLE NEEDLE-BEHANDLUNG**

OPEN-LOOP OR CLOSED-LOOP CONTROL DEVICE AND BLOOD-TREATMENT DEVICE FOR SINGLE-NEEDLE TREATMENT

DISPOSITIF DE COMMANDE OU DE RÉGLAGE ET DISPOSITIF DE TRAITEMENT DE SANG POUR UN TRAITEMENT À AIGUILLE UNIQUE

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorität: **10.04.2018 DE 102018108492**

(43) Veröffentlichungstag der Anmeldung:
**17.02.2021 Patentblatt 2021/07**

(73) Patentinhaber: **Fresenius Medical Care
Deutschland GmbH
61352 Bad Homburg (DE)**

(72) Erfinder:
• **KOPPERSCHMIDT, Pascal
97456 Dittelbrunn (DE)**
• **SCHLAEPER, Christian
61273 Wehrheim (DE)**

(74) Vertreter: **Bobbert & Partner
Patentanwälte PartmbB
Postfach 1252
85422 Erding (DE)**

(56) Entgegenhaltungen:
EP-A1- 0 115 835     EP-A1- 0 143 064
WO-A1-2010/121819     WO-A1-2015/173713
WO-A2-2009/006489     WO-A2-2009/127624
FR-A1- 2 252 106

**Beschreibung**

Die vorliegende Erfindung betrifft eine Steuer- oder Regelvorrichtung (hierin kurz: Steuervorrichtung) einer Blutbehandlungsvorrichtung gemäß Anspruch 1. Sie betrifft zudem eine Blutbehandlungsvorrichtung gemäß Anspruch 4.

Eine künstliche Einnadelniere ist in der EP 0 143 064 A1 offenbart.

Aus der Praxis sind Vorrichtungen zur extrakorporalen Behandlung von Blut bekannt, bei welcher Blut zur extrakorporalen Blutbehandlung über einen vaskulären Zugang mit nur einer Nadel (z. B. sog. "Single-Needle-Dialyse") dem Patienten entnommen und ihm wieder zurückgegeben wird.

Bei Single-Needle-Verfahren zur extrakorporalen Blutbehandlung wird zwischen einer sog. arteriellen Phase, bei der dem Patienten Blut über die z. B. eine Kanüle entnommen wird, und der sog. venösen Phase oder Rückgabe-Phase, bei der das Blut über dieselbe einzige Kanüle in das Gefäßsystem des Patienten zurückgegeben wird, unterschieden. Zwischen diesen beiden Phasen wird das Blut in einer Blutbehandlungseinrichtung, meist einem Blutfilter oder Dialysator, gereinigt. Bei den bekannten Vorrichtungen und Verfahren für die Single-Needle-Behandlung wird das Blut zwischen diesen Phasen ferner in einem Pufferbehälter, der im extrakorporalen Blutkreislauf (also auf der Blutseite) angeordnet ist, zwischengespeichert, um ein Umschalten zwischen den beiden Phasen zu ermöglichen.

Für das Single-Needle-Verfahren ist das Verwenden von nur einer Kanüle oder eines Singlelumen-Katheters, oder, allgemein ausgedrückt, nur einer Gefäßzugangseinrichtung, durch welche alternierend Patientenblut, z. B. aus einem, insbesondere kurzen, Shunt, zum Dialysegerät und nach der Reinigung zurück zum Patienten gefördert wird, bezeichnend. Das Verfahren kommt insbesondere in Notsituationen zum Einsatz.

Das Verfahren der Single-Needle-Behandlung ist beispielsweise in DRUKKER, PARSONS and MAHER, 5th Edition, Kluwer Academic Publishers, Dordrecht, 2004, Seite 365 mit mehr Details beschrieben.

Aufgabe der vorliegenden Erfindung kann sein, eine Steuervorrichtung und eine Blutbehandlungsvorrichtung anzugeben, die geeignet oder konfiguriert sind, das hierin offenbarte Verfahren auszuführen.

Die erfindungsgemäße Aufgabe wird gelöst durch eine Steuer- oder Regelvorrichtung mit den Merkmalen des Anspruchs 1, und durch eine Blutbehandlungsvorrichtung mit den Merkmalen des Anspruchs 4.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze.

Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wenn hierin von "programmiert" oder "konfiguriert" die Rede ist, so ist auch offenbart, diese Begriffe gegeneinander auszutauschen.

Die Angaben "oben" und "unten" sind hierin bei Zweifel des Fachmanns als absolute oder relative Raumangaben zu verstehen, welche sich auf die Ausrichtung des betreffenden Bauteils während seines üblichen Gebrauchs beziehen.

Der hierin offenbarte Blutschlauchsatz ist für eine Single-Needle-Behandlung geeignet.

Er umfasst eine Patientenschlauchleitung, die dazu vorgesehen oder angeordnet ist, um durch sie hindurch sowohl Blut aus dem Patientenblutkreislauf zu entnehmen und in den Blutschlauchsatz einzubringen als auch Blut aus dem Blutschlauchsatz heraus in den Patientenblutkreislauf rückzuführen.

Der hierin offenbarte Blutschlauchsatz umfasst ferner einen Y-förmigen Verbinder oder einen Drei-Wege-Verbinder. Der Verbinder ist mit der Patientenschlauchleitung verbunden.

Hierbei weist der Blutschlauchsatz allerdings keine blutseitige Single-Needle-Kammer auf oder ist auch mit keiner blutseitigen Single-Needle-Kammer, insbesondere in Fluidkommunikation, verbunden.

Wenn hierin von "blutseitig" die Rede ist, so bezeichnet dieser Ausdruck jene Abschnitte, durch welche bestimmungsgemäß bei der Blutbehandlung das zu behandelnde Blut extrakorporal strömt oder gepumpt wird, oder welche hiermit verbunden sind, etwa ein arterieller Druckmesser, ein optischer Dichtemesser, usw. Ein Blutschlauchsatz würde daher der Blutseite zugeordnet (da im Gebrauch von Blut durchströmt), während Leitungen, mittels welcher Dialysierflüssigkeit zum Blutfilter oder Dialysat vom Blutfilter weg gefördert werden, wie andere Abschnitte der Blutbehandlungsvorrichtung als maschinenseitig oder hydraulikseitig bezeichnet werden.

Wenn hierin von einer Single-Needle-Kammer die Rede ist, so ist hierunter eine Speichervorrichtung für ein vorübergehendes Speichern von Blut oder Vollblut zwischen einem Schritt des Entnehmens von Blut durch die Patientenschlauchleitung hindurch und dem darauffolgenden Schritt der Rückgabe durch die Patientenschlauchleitung hindurch zu verstehen. Ein alternativer Begriff für "Single-Needle-Kammer" ist beispielsweise "Compliancekammer". Diese Begriffe können hierin ausgetauscht werden.

Wenn hierin von einer Single-Needle-Kammer die Rede ist, so ist hierunter nicht ein Blutfilter oder ein Dialysator mit einer zumeist semi-permeablen Membran zu verstehen. Single-Needle-Kammern und Blutfilter oder Dialysatoren werden hierin als unterschiedliche, separate Komponenten verstanden.

Der hierin offenbarte Blutschlauchsatz kann ein extrakorporaler Blutkreislauf sein.

Die erfindungsgemäße Steuervorrichtung ist konfiguriert oder ausgestaltet zum Steuern oder Regeln einer Blutbehandlungsvorrichtung zum Behandeln von Blut mittels eines Single-Needle-Verfahrens.

Die Blutbehandlungsvorrichtung weist eine Blutpumpe auf, welche mit einem - beispielsweise erfindungsgemäßen - Blutschlauchsatz, beispielsweise wie oben beschrieben, verbindbar ist. Die Pumpe ist ausgestaltet zum Fördern von Blut durch den Blutschlauchsatz, wobei sie in einer Entnahmerichtung und vorzugsweise, insbesondere alternierend, auch in einer Rückgaberichtung (d. h. beispielsweise rückwärts) betreibbar ist.

Die zu steuernde Blutbehandlungsvorrichtung weist eine Rückgabepumpe auf. Die Rückgabepumpe kann beispielsweise die Blutpumpe sein, die zur Rückgabe z. B. in entgegengesetzter Richtung fördert. Alternativ oder ergänzend kann die Rückgabepumpe eine Druckluftquelle, eine Hydraulikpumpe, Dialysierflüssigkeitspumpe, Substituatpumpe oder jede andere Pumpe sein oder aufweisen. Die Rückgabepumpe ist optional angeordnet, um im Gebrauch der Blutbehandlungsvorrichtung Fluid in Richtung des Blutschlauchsatzes oder eines Abschnitts hiervon in einer Rückgaberichtung zu fördern. Alternativ ist die Rückgabepumpe optional mit dem erfindungsgemäßen Blutschlauchsatz oder einem Abschnitt hiervon verbindbar oder hiermit in Fluidkommunikation stehend ausgestaltet, um dem Fördern von Blut durch die Blutbehandlungseinrichtung und/oder durch den Blutschlauchsatz in einer Rückgaberichtung zu dienen.

Hierbei ist die Steuervorrichtung konfiguriert oder programmiert, um mittels der Blutbehandlungsvorrichtung ein Verfahren auszuführen, welches die folgenden Schritte a) und b) mehrfach alternierend umfasst:

a) Die Blutpumpe wird in einer Entnahmerichtung betrieben, in welcher Blut aus dem Patientenblutkreislauf entnommen und entlang einer Patientenschlauchleitung in den erfindungsgemäßen Blutschlauchsatz gepumpt wird. Sie entnimmt dem Patienten in diesem Schritt also Blut. Das Volumen des in diesem Schritt entnommenen Bluts kann z. B. zwischen 30 ml und 100 ml betragen.

b) Die Rückgabepumpe wird in einer Rückgaberichtung betrieben. Dadurch oder dabei wird Blut aus dem Blutschlauchsatz herausgepumpt, es wird hierbei entlang der Patientenschlauchleitung in den Patientenblutkreislauf zurück gepumpt. Das Volumen des in diesem Schritt dem Patienten zurückgegebenen ("re-infundierten") Bluts beträgt zwischen 30 ml und 100 ml. Das Volumen soll - zum Erzielen einer ausgeglichenen Volumen- oder Massenbilanz - vorzugsweise dem in Schritt a) entnommenen Blutvolumen entsprechen. Nach Abschluss des Schrittes b) kann ein neuer Zyklus der Schritte a) und b) durch Beginnen eines weiteren Schrittes a) begonnen werden.

Dabei wird das Blut zwischen seiner Entnahme in Schritt a) und seiner Rückgabe in Schritt b) nicht in einer blutseitigen Single-Needle-Kammer gespeichert.

Die erfindungsgemäße Blutbehandlungsvorrichtung, die zum Durchführen einer Single-Needle-Blutbehandlung geeignet ist, weist eine Blutpumpe auf, welche mit einem Blutschlauchsatz, beispielsweise wie oben beschrieben, verbindbar ist. Die Pumpe ist ausgestaltet zum Fördern von Blut durch den Blutschlauchsatz, wobei sie in einer Entnahmerichtung und vorzugsweise, z. B. alternierend, auch in einer Rückgaberichtung betreibbar ist.

Die Blutbehandlungsvorrichtung weist eine Rückgabepumpe auf. Die Rückgabepumpe kann beispielsweise die Blutpumpe sein, die zur Rückgabe z. B. in entgegengesetzter Richtung fördert. Alternativ oder ergänzend kann die Rückgabepumpe eine Druckluftquelle, eine Hydraulikpumpe, Dialysierflüssigkeitspumpe, Substituatpumpe oder jede andere Pumpe sein. Die Rückgabepumpe ist optional angeordnet, um im Gebrauch der Blutbehandlungsvorrichtung Fluid in Richtung des Blutschlauchsatzes oder eines Abschnitts hiervon in einer Rückgaberichtung zu fördern. Alternativ ist die Rückgabepumpe mit dem erfindungsgemäßen Blutschlauchsatz oder einem Abschnitt hiervon verbindbar oder hiermit in Fluidverbindung stehend ausgestaltet, um zum Fördern von Blut durch den Blutschlauchsatz in einer Rückgaberichtung zu dienen oder beizutragen.

Des Weiteren weist die erfindungsgemäße Blutbehandlungsvorrichtung eine Steuer- oder Regelvorrichtung (kurz: Steuervorrichtung) auf, welche dazu konfiguriert oder programmiert ist, die Blutbehandlungsvorrichtung zu steuern oder zu regeln. Hierbei ist die Steuervorrichtung konfiguriert oder programmiert, um mittels der Blutbehandlungsvorrichtung ein Verfahren auszuführen, welches die folgenden Schritte a) und b), die auch als arterielle Phase bzw. venöse Phase bezeichnet werden können, mehrfach alternierend umfasst:

a) Die Blutpumpe wird in einer Entnahmerichtung betrieben, in welcher Blut aus dem Patientenblutkreislauf entnommen und entlang einer Patientenschlauchleitung in den hierin offenbarten Blutschlauchsatz gepumpt wird. Sie entnimmt dem Patienten in diesem Schritt also Blut. Das Volumen des in diesem Schritt entnommenen Bluts kann z. B. zwischen 30 ml und 100 ml betragen.

b) Die Rückgabepumpe wird in einer Rückgaberichtung betrieben. Dadurch oder dabei wird Blut aus dem Blutschlauchsatz heraus und entlang der Patientenschlauchleitung in den Patientenblutkreislauf zurück gepumpt. Das Volumen des in diesem Schritt dem Patienten zurückgegebenen ("re-infundierten") Bluts beträgt zwischen 30 ml und 100 ml. Das Volumen wird dem in Schritt a) entnommenen Blutvolumen entsprechen. Ein neuer Zyklus aus Entnahme und Rückgabe kann sich anschließen.

Dabei wird das Blut zwischen seiner Entnahme in Schritt a) und seiner Rückgabe in Schritt b) nicht in einer blutseitigen Single-Needle-Kammer gespeichert.

Das hierin offenbarte Verfahren zum Reinigen von Blut mittels eines Single-Needle-Verfahrens wird mittels einer erfindungsgemäßen Blutbehandlungsvorrichtung durchgeführt.

Beim hierin offenbarten Verwenden eines Blutschlauchsatzes zur Single-Needle-Behandlung, insbesondere im Rahmen eines der hierin beschriebenen Blutbehandlungsverfahren, wird Blut nicht in einer blutseitigen Single-Needle-Kammer bzw. einer blutseitigen Compliancekammer zwischengespeichert, also nicht in einer Single-Needle-Kammer bzw. Compliancekammer, die Teil des Blutschlauchsatzes und/oder auf der Blutseite vorgesehen ist.

Erfindungsgemäße Ausführungsformen können aufbauend auf einem der unabhängigen Ansprüche eines oder mehrere der vorstehend oder im Folgenden genannten Merkmale aufweisen. Dabei können die hierin genannten Merkmale in beliebiger Kombination Gegenstand von erfindungsgemäßen Ausführungsformen aufbauend auf einem der unabhängigen Ansprüche sein, sofern der Fachmann eine konkrete Kombination nicht als technisch unmöglich erkennt.

Wann immer hierin von einer Ausführungsform die Rede ist, so handelt es sich um eine beispielhafte, erfindungsgemäße Ausführungsform, aufbauend auf einem der unabhängigen Ansprüche.

In manchen Ausführungsformen wird Blut nicht in einer maschinenseitigen Single-Needle-Kammer bzw. einer maschinenseitigen Compliancekammer zwischengespeichert, also nicht in einer Single-Needle-Kammer bzw. Compliancekammer, die Teil der Hydraulik und/oder auf der Hydraulikseite vorgesehen ist.

In einigen Ausführungsformen weist der Blutschlauchsatz neben einer Patientenschlauchleitung keine weitere Patientenschlauchleitung auf, die mit dem Gefäßkreislauf des Patienten und/oder mit einer Zugangseinrichtung verbunden wäre, sondern insgesamt nur eine.

In manchen Ausführungsformen weist der Blutschlauchsatz genau eine Zugangseinrichtung zum Herstellen eines Zugangs zwischen der Patientenschlauchleitung und dem Patientenblutkreislauf auf.

Eine Zugangseinrichtung kann beispielsweise eine Nadel, ein Verbinder usw. sein und ist vorzugsweise einlumig.

In manchen Ausführungsformen kann die Membran eine hohe Membranpermeabilität (High Flux) aufweisen.

In einigen Ausführungsformen umfasst das erfindungsgemäße Verfahren ebenso wie das durch die Steuervorrichtung ausgeführte Verfahren optional ferner das Schließen einer venösen, also stromab der Blutbehandlungseinrichtung (Blutfilter, Dialysator, usw.) gelegenen Klemme oder Schlauchklemme oder ein anderweitiges Unterbinden des Blutstroms stromab der Blutbehandlungseinrichtung, während oder bevor Blut im oben genannten Schritt a) vom Patienten in Richtung zum Blutfilter gefördert wird.

Weiter wird mittels der Steuervorrichtung der Übertritt von Volumen, wie z. B. Plasma oder Blutwasser des geförderten Bluts, über die semi-permeable Membran des Blutfilters durch Aufbauen eines Druckunterschieds, z. B. eines Druckgefälles zwischen der Blutkammer und der Dialysierflüssigkeitskammer, bewirkt. Dies kann beispielsweise durch aktive hydraulikseitige Ultrafiltration mittels einer oder mehrerer Ultrafiltrationspumpen und/oder passiv durch Volumenverschiebung unter Abbau der durch die Blutpumpe im Blutfilter erzeugten Druckerhöhung unterstützt werden oder erfolgen.

Schließlich umfasst das durch die Steuervorrichtung ausgeführte Verfahren in einigen Ausführungsformen das Öffnen der venösen Schlauchklemme oder das Beenden des anderweitigen Unterbindens.

Alternativ oder ergänzend, umfasst in manchen Ausführungsformen das Verfahren oder das durch die Steuervorrichtung ausgeführte Verfahren das Bewirken des Übertritts von Flüssigkeit aus der Dialysierflüssigkeitskammer über die semi-permeable Membran in die Blutkammer des Blutfilters. Dies kann beispielsweise durch Aufbauen eines geeigneten Drucks auf der Hydraulikseite oder der Dialysierflüssigkeitsseite (Dialysierseite) geschehen, während im oben genannten Schritt b) entlang der Patientenschlauchleitung Blut in den Patientenblutkreislauf zurück gepumpt wird.

In manchen Ausführungsformen erfolgt z. B. in diesem Schritt optional die Zugabe von Substituatflüssigkeit über eine Substituatzugabestelle des Blutschlauchsatzes, z. B. über einen Konnektor und z. B. aus einer Substituatquelle, anstelle der Rückgabe von Fluid über die Membran hinweg oder ergänzend hierzu. Dies kann dem Verdrängen von Blut und dem Rückführen von Blut zum Patienten dienen oder hierbei unterstützend wirken. Eine Volumenverschiebung in Schritt b) über die Membran des Blutfilters hinweg kann in diesen Ausführungsformen daher unterbleiben oder ergänzt werden.

Das Verfahren umfasst ein Steuern und/oder Überwachen des Verfahrens durch Analyse von Druckmesswerten, insbesondere des arteriellen und/oder venösen Drucksensors, und/oder der Motorstromaufnahme mindestens einer der hierin genannten Pumpen.

In manchen Ausführungsformen wird der Schritt a) beendet, wozu entweder die Entnahmetätigkeit der Blutpumpe gestoppt oder verringert oder (alternativ oder ergänzend) die Rückgabetätigkeit der Rückgabepumpe gestartet oder erhöht wird, wenn beispielsweise der Transmembrandruck einen für die Phase des Schrittes a) vorbestimmten Wert erreicht oder überschritten hat. Dieser Wert kann höher liegen als andere Werte, die während anderer Phasen der Blutbehandlung für den Transmembrandruck als Grenzwert vorab festgelegt wurden. Ist der für den Schritt a) vorbestimmte Wert erreicht/überschritten, so gilt eine maximal erwünschte oder zulässige Hämokonzentration des sich in der Blutkammer des Blutfilters befindenden Bluts als erreicht. Die erfindungsgemäßen Vorrichtungen können zum Ausführen oder Bewirken dieser Ausführung - wie auch jeder anderen hiermit offenbarten Ausführungsform - konfiguriert sein.

In einigen Ausführungsformen umfasst das Verfahren eine zwischen dem Schritt a) (z. B. dessen Ende) und dem Schritt b) (z. B. dessen Beginn) eingefügte, hierin als Interimsphase oder Schritt c) bezeichnete, Phase. Während dieser kann die Blutpumpe und optional auch die Rückgabepumpe gestoppt oder deutlich verlangsamt sein. Während dieser Interimsphase kann optional die venöse Schlauchklemme geschlossen sein. Das in der Blutkammer vorliegende Blut kann zum Verweilen in der Blutkammer gezwungen sein. Während dieser Interimsphase kann die Dialysierflüssigkeitskammer des Blutfilters von Dialysierflüssigkeit durchströmt werden. Die Zeitdauer, während welcher auf diese Weise gespült wird, kann z. B. mehr als 1 Sekunden , insbesondere zwischen 1 und 30 Sekunden betragen. Die Zeit, während welcher auf diese Weise gespült wird, kann auf jene Zeit begrenzt sein (oder dieser entsprechen), welche vergeht bis ein vorbestimmtes Volumen an Dialysierflüssigkeit die Dialysierflüssigkeitskammer des Blutfilters durchströmt hat. Dieses Volumen kann z. B. auf das 1 - 3fache des Flüssigkeitsvolumens der Dialysierflüssigkeitskammer festgelegt sein.

Das Einschieben oder Vorsehen einer solchen Interimsphase kann die Effizienz der Blutreinigung erhöhen, da während dieser Phase frische Dialysierflüssigkeit an konzentriertem Blut, durch die semi-permeable Membran getrennt, vorbeigeführt wird. Es ist das Ausnutzen des Zustands hoher Konzentration während der Interimsphase, das zur erhöhten diffusiven Begrenzung des Bluts von Urämietoxinen mittels Filtration und damit zur vorstehenden Effizienzsteigerung führt.

In manchen Ausführungsformen ist die vorstehend beschriebene Interimsphase nicht streng derart zwischen die Entnahmephase und die Rückgabephase eingefügt, dass entweder die Entnahmephase, die Interimsphase oder die Rückgabephase abliefe. Zwar ist genau dies in manchen Ausführungsformen der Fall, in anderen Ausführungsformen überschneiden sich jedoch stets zwei der vorgenannten Phasen, also entweder die Entnahmephase mit der Interimsphase (die dann vermutlich nicht mehr als "interim" bezeichnet werden kann), oder die Interimsphase mit der Rückgabephase.

Dabei kann die Interimsphase optional gar nicht mehr alleine vorliegen, sondern geht während ihrer gesamten Dauer mit Entnahmeströmen oder mit Rückgabeströmen einher.

So kann in einigen Ausführungsformen vorgesehen sein, bereits während der hierin als Schritt a) bezeichneten Phase der Entnahme mittels der auf der Hydraulikseite der Blutbehandlungsvorrichtung vorgesehenen Pumpen (eine stromauf der Dialysierflüssigkeitskammer des Blutfilters gelegen, die andere stromab dieser Kammer), in der Dialysierflüssigkeitskammer sowohl einen (bezogen auf die Blutkammer) niedrigeren Druck oder Unterdruck zu erzeugen, als auch gleichzeitig einen Dialysierflüssigkeitsstrom durch die Dialysierflüssigkeitskammer aufrechtzuerhalten. Auf diese Weise kann auch bereits in Schritt a) sowohl eine Filtration als auch eine Dialyse, also eine Blutreinigung sowohl über Konvektion als auch über Diffusion, erfolgen.

Ebenso kann, ergänzend oder alternativ, in manchen Ausführungsformen vorgesehen sein, während der hierin als Schritt b) bezeichneten Phase der Rückgabe mittels vorgenannter Pumpen in der Dialysierflüssigkeitskammer sowohl einen (bezogen auf die Blutkammer) höheren Druck oder Überdruck zu erzeugen, als auch gleichzeitig einen Dialysierflüssigkeitsstrom durch die Dialysierflüssigkeitskammer aufrechtzuerhalten. Auf diese Weise kann auch bereits in Schritt b) sowohl eine Volumensubstitution als auch eine Dialyse erfolgen.

In einigen Ausführungsformen ist die Steuerungsvorrichtung konfiguriert, um die vorgenannten Pumpen, wie vorstehend beschrieben, zu steuern oder zu regeln, in Abhängigkeit von der Steuerung der Blutpumpe, der Rückgabepumpe und/oder Ventilen oder Klemmen wie z. B. der venösen Schlauchklemme. So kann z. B. programmiert oder konfiguriert sein, den Dialysierflüssigkeitsstrom entlang der semi-permeablen Membran (ohne durch sie hindurchzutreten) zu starten, nachdem der bewirkte Übertritt von Plasmawasser über die semi-permeable Membran abgeschlossen ist, etwa weil die Blutpumpe gestoppt oder die venöse Schlauchklemme geöffnet wird oder wurde. Ebenso kann konfiguriert sein, den Dialysierflüssigkeitsstrom entlang der Membran zu beenden, sobald mit dem Übertritt der frischen Dialysierflüssigkeit oder Substituat oder einer anderen Flüssigkeit für die Infusion in den Patienten über die Membran auf die Blutseite begonnen werden soll.

In einigen Ausführungsformen ist die Blutbehandlungsvorrichtung als Hämodialysevorrichtung, Hämofiltrationsvorrichtung oder als Hämodiafiltrationsvorrichtung ausgestaltet, insbesondere als eine Vorrichtung für die chronische Nierenersatztherapie oder für die kontinuierliche Nierenersatztherapie (CRRT = *continuous renal replacement therapy*).

Die Blutbehandlungsvorrichtung kann als Vorrichtung zur Akutbehandlung ausgestaltet sein, und Dialysierflüssigkeit kann beispielsweise mittels Beutel zur Verfügung gestellt werden.

Die Blutbehandlungsvorrichtung kann als Vorrichtung zur Dauerbehandlung oder zur Behandlung dauerhaft dialysepflichtiger Patienten ausgestaltet sein, und Dialysierflüssigkeit kann beispielsweise von der Behandlungsvorrichtung online gemischt werden.

In einigen Ausführungsformen ist die Blutpumpe zugleich die Rückgabepumpe. Die Blutpumpe kann alternierend in einer Entnahmerichtung und in einer Rückgaberichtung betrieben werden. Alternativ oder ergänzend kann das Öffnen und Schließen von Fluidwegen innerhalb des Blutschlauchsatzes dazu führen, dass Blut auch bei Betreiben nur einer Pumpe, z. B. der Blutpumpe, mal dem Patienten entnommen und mal dem Patienten zurückgegeben wird.

In einigen Ausführungsformen ist keine Single-Needle-Kammer bzw. Compliancekammer vorgesehen, auch nicht auf der Seite der Hydraulik. Insbesondere ist keine Single-Needle-Kammer bzw. Compliancekammer vorgesehen, die als Oszillationsvolumen oder Volumen zum Aufnehmen und nachfolgenden Abgeben von Fluid zwischen der Dialysierflüssigkeitskammer und einer Abfallpumpe oder einer Ultrafiltrationspumpe vorgesehen ist und mit einer Leitung zwischen Dialysierflüssigkeitskammer und einer Abfallpumpe oder einer Ultrafiltrationspumpe, insbesondere mittels Stichleitung, verbunden ist, oder hiervon abzweigt.

Unter einer Compliancekammer wird hierin nicht etwa ein Volumen verstanden, welches sich ergibt oder entsteht, wenn elastische Fluidleitungen, etwa die arterielle Blutleitung oder die venöse Blutleitung, sich bei erhöhtem Leitungsinnendruck über ihr Nominal- oder Ruhevolumen hinaus weiten und somit mehr Volumen als zuvor aufnehmen können. Zwar wird dieser Effekt üblicherweise ebenfalls als "Compliance" (der Leitungen) bezeichnet, letzteres beschreibt allerdings nur ein Verhalten des Materials der ohnehin vorliegenden Leitung. Eine "Compliancekammer", so wie dieser Begriff hierin verwendet wird, ist dieses Materialverhalten hingegen nicht. Eine "Compliancekammer", wäre vielmehr eine Kammer, die als solche erkennbar ist, und die optionaler Weise keine andere Funktion als das Zwischenspeichern von Fluid hat. Sie kann in Fluidverbindung mit einer Leitung stehen, z. B. in diese fluidisch eingeschaltet oder mittels Stichleitung mit dieser verbunden sein. Eine Compliancekammer ist aber optional erkennbar als solche ausgestaltet und/oder unterscheidet sich von der sie speisenden Leitung. Würde man sich die Compliancekammer wegdenken, so bliebe optional die sie speisende Leitung übrig.

In einigen Ausführungsformen weist die Blutbehandlungsvorrichtung eine Förderpumpe zum Fördern von frischer Dialysierflüssigkeit in Richtung zur Dialysierflüssigkeitskammer und eine Abfallpumpe zum Fördern von verbrauchtem Dialysat in Richtung weg von der Dialysierflüssigkeitskammer auf.

Manche oder alle erfindungsgemäßen Ausführungsformen können einen, mehrere oder alle der oben und/oder im Folgenden genannten Vorteile aufweisen.

In herkömmlichen, vollständig luftfreien extrakorporalen Blutschlauchsystemen fehlt die Möglichkeit, das Patientenblut während der o. g. Phasenwechsel (Übergang zwischen Entnahme und Rückgabe) zwischenzulagern. Über zu- und abschaltbare Single-Needle-Kammern lässt sich zwar auch in solchen Systemen das Single-Needle-Verfahren umsetzen, dies erfordert jedoch eine aufwändige Mimik zur phasensynchronisierten Bereitstellung eines bedarfsgerechten Speichervolumens. Die erforderliche Steuerung der Blutbehandlungsvorrichtung ist somit vergleichsweise aufwändig. Ferner kann das benötigte Blutschlauchsystem deutlich komplexer sein als z. B. beim Double-Needle-Verfahren, bei dem Blut über zwei Gefäßzugänge kontinuierlich entnommen bzw. rückgeführt wird. Mit Hilfe der hierin beschriebenen Erfindung lässt sich vorteilhafterweise sowohl auf die aufwändige Mimik zur phasensynchronisierten Bereitstellung des Speichervolumens als auch auf eine aufwändige Steuerung sowie auf einen aufwändigen Blutschlauchsatz verzichten.

Die Blutreinigung kann vorteilhafterweise dennoch durch die Kombination eines diffusiven und konvektiven Stoffaustauschs erfolgen.

Da das extrakorporale Blutschlauchsystem komplett luftfrei bleiben kann, werden Blut/Luft-Kontakte und die damit einhergehende Koagulationsgefahr vorteilhaft reduziert oder sogar vermieden. Blut/Luft-Kontakte führen ebenso wie Blutstagnationen zu verstärkter Neigung zur Thrombenbildung im Blutschlauchsystem, was den verstärkten Einsatz von Antikoagulantien erfordert. Erfindungsgemäß erhöht sich somit die Sicherheit für den behandelten Patienten. Zudem können vorteilhaft Antikoagulantien eingespart werden und/oder deren Einsatz verringert werden.

Ein weiterer Vorteil besteht darin, dass neben den üblichen Pumpen, wie z. B. der Blutpumpe oder der Ultrafiltrationspumpe, kein weiterer Aktor im extrakorporalen Blutkreislauf notwendig ist. Das Verfahren kann daher bei bestehenden Gerätesystemen mit wenig Aufwand implementiert werden.

Die vorliegende Erfindung wird im Folgenden anhand der beigefügten Zeichnung, in welcher gleiche Bezugszeichen gleiche oder ähnliche Bauteile bezeichnen, exemplarisch erläutert. In den Figuren gilt:

**Fig. 1**  zeigt in vereinfachter Darstellung eine erfindungsgemäße Blutbehandlungsvorrichtung mit einem extrakorporalen Blutkreislauf in einer ersten Ausführungsform;

**Fig. 2a**  zeigt exemplarisch in graphischer Darstellung den schematischen Aufbau des hierin offenbarten Blutschlauchsatzes im Gebrauch während einer Entnahmephase; und

**Fig. 2b** zeigt exemplarisch in graphischer Darstellung den schematischen Aufbau des Blutschlauchsatzes der Fig. 2a während einer Rückgabephase; und

**Fig. 2c** zeigt exemplarisch in graphischer Darstellung den schematischen Aufbau des Blutschlauchsatzes der Fig. 2a und 2b während einer optionalen Zwischen- oder Interimsphase.

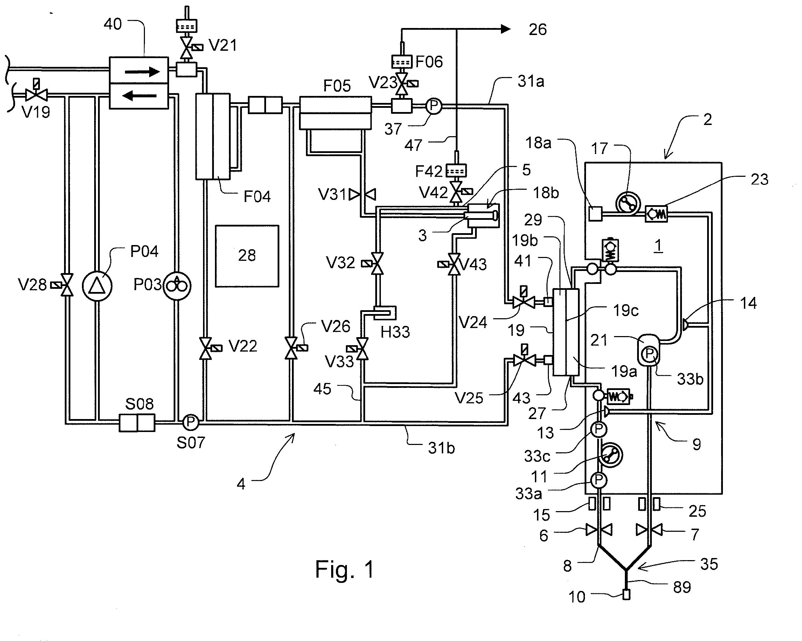

**Fig. 1** zeigt in vereinfachter Darstellung eine exemplarische Ausführungsform einer erfindungsgemäßen Blutbehandlungsvorrichtung 4, verbunden mit einem Blutschlauchsatz oder extrakorporalen Blutkreislauf 1. Die Blutbehandlungsvorrichtung 4 der Fig. 1 ist eine Vorrichtung zur Behandlung dauerhaft dialysepflichtiger Patienten. Die erfindungsgemäße Blutbehandlungsvorrichtung 4 ist hierauf jedoch nicht beschränkt.

Der extrakorporale Blutkreislauf 1 verläuft rein exemplarisch außerhalb und innerhalb einer Blutkassette 2 und verbindet diese fluidisch mit der Behandlungsvorrichtung 4. Der extrakorporale Blutkreislauf 1 weist genau eine Zugangseinrichtung 10, beispielsweise eine Konnektionsnadel, auf oder ist hiermit verbunden. Ein Fluidstrom durch den extrakorporalen Blutkreislauf 1 oder durch Abschnitte hiervon kann mittels einer arteriellen Schlauchklemme 6, angeordnet in dessen arterieller Blutleitung 8, unterbunden werden, ferner mittels einer venösen Schlauchklemme 7, angeordnet in dessen venöser Blutleitung 9.

Ein Abschnitt des extrakorporalen Blutkreislaufs 1 ist in eine Blutpumpe 11 der Blutbehandlungsvorrichtung 4 eingelegt. Der extrakorporale Blutkreislauf 1 weist optional eine Zugabestelle 13 für Substituatflüssigkeit (in Prädilution) und optional eine Zugabestelle 14 für Substituatflüssigkeit (in Postdilution) auf. Die Zugabestellen 13 und 14 sind hier exemplarisch als Phantomventile ausgeführt. Phantomventile dieser Art sind in der WO 2010/121819 A1 der Anmelderin auch der vorliegenden Erfindung beschrieben. Für Details wird auf jene Offenlegungsschrift verwiesen.

Ein optionaler arterieller Luft-Blut-Detektor 15 ist optional an der arteriellen Blutleitung 8 vorgesehen.

Fig. 1 zeigt ferner optional eine Substituatpumpe 17 der Blutbehandlungsvorrichtung 4. Diese liegt stromabwärts einer Verbindungsstelle, an welcher optional die Blutkassette 2 vor deren Gebrauch mit ihrem Substituatport 18a mit einem optionalen, automatischen Substituatkonnektor 18b der Blutbehandlungsvorrichtung 4 verbunden werden kann. Der automatische Substituatkonnektor 18b weist im Beispiel der Fig. 1 optional eine erste Fluidführung 3, eine zweite Fluidführung 5 und eine dritte Fluidführung zum Spülen des automatischen Substituatkonnektors 18b und zum Leiten von Substituat durch den automatischen Substituatkonnektor 18b hindurch auf.

In den extrakorporalen Blutkreislauf 1 ist ein Blutfilter 19 mit einer Blutkammer 19a und einer

Dialysierflüssigkeitskammer 19b eingeschaltet. Blutkammer 19a und Dialysierflüssigkeitskammer 19b sind durch eine semi-permeable Membran 19c voneinander getrennt.

Die Blutkassette 2 weist optional eine venöse Luftabscheidekammer 21 auf.

Eine Substituatleitung der Blutkassette 2 weist optional ein Rückschlagventil 23 auf.

Der extrakorporale Blutkreislauf 1 weist optional einen venösen Luft-Substituatflüssigkeit-Blut-Detektor 25 an der venösen Blutleitung 9 auf.

Die Blutbehandlungsvorrichtung 4 weist optional eine Druckluftquelle 26 auf oder ist hiermit verbunden.

In Fig. 1 sind ferner eine Dialysierflüssigkeitszulaufleitung 31a, welche Dialysierflüssigkeit zur Dialysierflüssigkeitskammer 19b führt, und eine Dialysatablaufleitung 31b, welche Dialysat von der Dialysierflüssigkeitskammer 19b wegführt, zu erkennen.

Ein Drucksensor 33a ist optional stromaufwärts der Blutpumpe 11 in der arteriellen Blutleitung 8 vorgesehen.

Ein Drucksensor 33b ist optional im Bereich der venösen Luftabscheidekammer 21 in der venösen Blutleitung 9 vorgesehen.

Ein Drucksensor 33c, auch als Präfilterdrucksensor bezeichnet, ist optional stromabwärts der Blutpumpe 11 in der arteriellen Blutleitung 8 vorgesehen. Dieser kann stromaufwärts der Zugabestelle 13 angeordnet sein.

Ein wiederum weiterer Drucksensor 37 befindet sich optional in oder an der Dialysierflüssigkeitszulaufleitung 31a zwischen der Druckquelle 26 und dem Blutfilter 19.

Ventile V19, V22, V24, V25, V26, V28, V31, V32 und V33 sind optional und in Abschnitten der Hydraulik der Blutbehandlungsvorrichtung 4 vorgesehen.

Das Ventil V24 liegt in oder an der Dialysierflüssigkeitszulaufleitung 31a.

Das Ventil V25 liegt in oder an der Dialysatablaufleitung 31b.

Das Ventil V28 liegt in einer Dialysatablaufleitung 31b.

Das Ventil V31 liegt in der ersten Fluidführung 3 des automatischen Substituatkonnektors 18b oder in einer zu diesem hin führenden Leitung.

Das Ventil V32 liegt in der zweiten Fluidführung 5 des automatischen Substituatkonnektors 18b oder in einer zu diesem hinführenden Leitung.

Das Ventil V33 liegt in der Drain-Leitung 45.

Das Ventil V19 liegt stromabwärts aller vorstehend genannten Ventile V24, V25, V28, V31, V32 und V33.

Gezeigt ist weiter eine Ultrafiltrationspumpe P03. Nur angedeutet ist eine Hydraulik-Bilanzkammer 40.

Eine erfindungsgemäße Steuer- oder Regelvorrichtung 28 zum Steuern oder Regeln der Blutbehandlungsvorrichtung 4 ist vorgesehen und kann mit allen o. g. Komponenten der Blutbehandlungsvorrichtung 4 in Signal- und/oder Steuerverbindung stehen.

Üblicherweise wird im Stand der Technik das extrakorporal geförderte Patientenblut bei einer Single-Needle-Behandlung vor oder nach seiner Reinigung in der Blutbehandlungseinrichtung in einer Compliancekammer des extrakorporalen Schlauchsystems zwischengeparkt, so dass die alternierende Förderung möglich wird. In der ersten der beiden alternierenden Phasen wird durch den einen Zugang das Patientenblut mittels der Blutpumpe 11 in die blutseitige Compliancekammer gefördert, wobei der Reinigungsprozess noch nicht oder bereits abgeschlossen ist. In der zweiten Phase wird das Blut aus der Compliancekammer (zumeist mittels eines neben der Blutpumpe 11 weiteren Aktors oder Pumpe) zum Patienten zurückgepumpt. Dieser Aktor kann eine zweite Pumpe oder ein Kompressor, z. B. für Luft, sein. Das Volumen der Compliancekammer entspricht dem Single-Needle-Schlagvolumen und beträgt allgemein zwischen 25 und 100 ml, gelegentlich zwischen 30 und 60 ml. Eine solche herkömmliche, blutseitige Compliancekammer des extrakorporalen Schlauchsystems oder Blutschlauchsatzes ist erfindungsgemäß nicht erforderlich und nicht vorgesehen.

Was Fig. 1 nicht zeigt ist die optionale, ebenfalls erfindungsgemäße Ausführungsform, bei welcher eine Single-Needle-Kammer bzw. Compliancekammer auf der Hydraulikseite, als Teil der Hydraulik oder auf Seiten der Blutbehandlungsvorrichtung 4 (also nicht im Blutschlauchsatz 1 oder nicht hiermit körperlich verbunden) vorgesehen ist. Eine solche Single-Needle-Kammer bzw. Compliancekammer wäre bezogen auf die Darstellung der Fig. 1 also links vom Blutfilter 19, jedenfalls links von der semi-permeablen Membran 19c, vorgesehen.

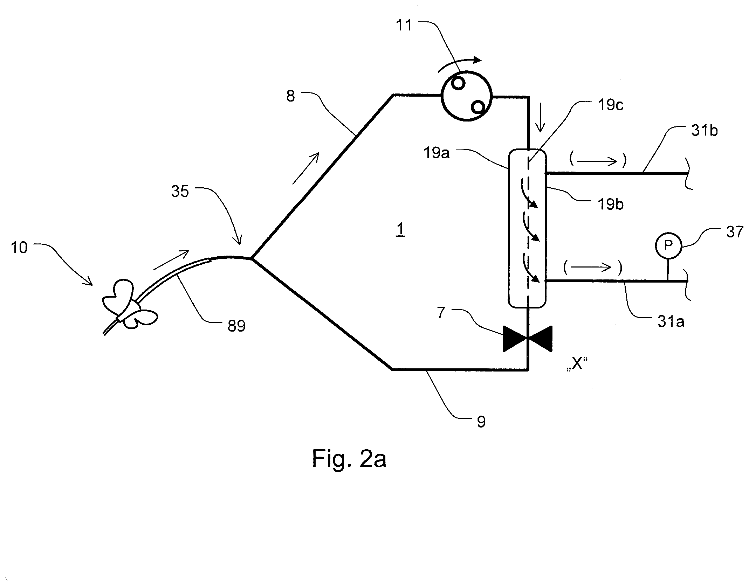

**Fig. 2a** zeigt exemplarisch in graphischer Darstellung den schematischen Aufbau des Blutschlauchsatzes 1 während der oben mit a) bezeichneten ersten Phase des hierin offenbarten Verfahrens.

Das anhand von Fig. 2a beschriebene Verfahren nutzt das Volumen der Dialysierflüssigkeitskammer 19b und ggf. der hieran angeschlossenen Leitungen der Hydraulik der Blutbehandlungsvorrichtung 4 zum Aufnehmen von flüssigen Anteilen von Blut, vorzugsweise gefolgt durch deren Verwerfen in einen Ausguss, in einen Beutel oder dergleichen. Bei den angeschlossenen Leitungen kann es sich um die Dialysierflüssigkeitszulaufleitung 31a und/oder die Dialysatablaufleitung 31b handeln.

Erfindungsgemäß wird durch die semi-permeable Membran 19c kein Vollblut in die Hydraulik gepumpt und über diese verworfen, sondern Plasmawasser. Dieses kann als Effluent oder Filtrat verworfen werden (z. B. in den Ausguss). Während des Überführens von Plasmawasser auf die Hydraulikseite, was in der ersten Phase (oben als Schritt a) bezeichnet) erfolgt, wird das Blut in der Blutkammer 19a des Blutfilters 19 hämokonzentriert. Das Plasmawasser wird über die semi-permeable Membran 19c des Blutfilters 19 verschoben, indem bei geschlossener venöser Schlauchklemme 7 und in Richtung zum Blutfilter 19 pumpender Blutpumpe 11 ein erhöhter Druck in der Blutkammer 19a aufgebaut wird. Dieser ist höher als jener, der in der Dialysierflüssigkeitskammer 19b, also auf der Dialysierflüssigkeitsseite des Blutfilters 19, herrscht. Die sich ergebenden Flüssigkeitsströme sind über die Membran 19c hinweg mittels Pfeile angedeutet. Sie sind ferner mittels Pfeile, die in Klammern gesetzt sind, an den Leitungen 31a und 31b angedeutet. Die Klammern sollen hier andeuten, dass es keinesfalls zu einem oder mehreren der Flüsse, deren Pfeile in Klammern stehen, kommen muss, optional jedoch kommen kann.

Die Blutpumpe 11 fördert offensichtlich, so dass es zu Flüssen auch in der Zugangseinrichtung 89 und in der arteriellen Blutleitung 8 in Richtung zum Blutfilter 19 kommt. Die venöse Schlauchklemme 7 ist dabei geschlossen, was mit einem "X" neben der Schlauchklemme 7 angedeutet ist. In der venösen Blutleitung 9, die sich dem Blutfilter 19 stromab anschließt, fließt aufgrund der geschlossenen Schlauchklemme 7 kein Blut.

Korpuskulare Bestandteile, die die semi-permeable Membran 19c nicht überwinden können, verbleiben auf der Blutseite des Blutfilters 19, also auf Seite der Blutkammer 19a, was dort zur o. g. Konzentrierung führt.

Es ist erkennbar, dass der in Fig. 2a verwendete, Blutschlauchsatz 1 keine blutseitige Single-Needle-Kammer aufweist. Er ist jedoch mit einem Blutfilter 19 verbunden, und er wird zur Single-Needle-Behandlung verwendet.

Der in der in Fig. 2a gezeigten Phase stattfindende Übertritt von Fluid über die semi-permeable Membran 19c, also von der Blutkammer 19a in die Dialysierflüssigkeitskammer 19b kann als "positive Ultrafiltration" bezeichnet werden. Sie kann zumindest phasenweise mit einer akzeptierten Fehlbilanz einhergehen. Die Fehlbilanz kann sich z. B. dadurch ergeben, dass Fluid aus der Hydraulikseite oder Dialysierseite (also rechts der semi-permeablen Membran 19c in Fig. 2a) verworfen wird, z. B. in einen Ausguss, z. B. über die in Fig. 1 gezeigte Drain-Leitung 45.

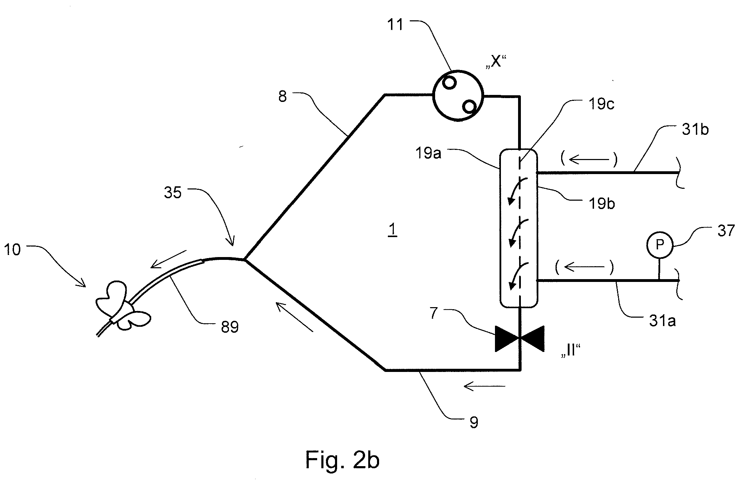

**Fig. 2b** zeigt in der Darstellung der Fig. 2a exemplarisch den Blutschlauchsatz 1 während der zweiten Phase des Verfahrens.

In der zweiten Phase (oben als Schritt b) bezeichnet) wird ein Flüssigkeitsvolumen, das quantitativ optional dem zuvor über die semi-permeable Membran 19c verschobenen Flüssigkeitsvolumen entspricht, über die semi-permeable Membran 19c und in den Blutschlauchsatz 1 gedrückt. Die in Schritt b) verschobene Flüssigkeit ist zumeist frische Dialysierflüssigkeit oder Substituatlösung, die etwa aus einem Beutel stammt oder von der Blutbehandlungsvorrichtung hergestellt ist. Das Volumen auf der Blutseite des Blutfilters 19 beträgt optional ca. 100-150 ml.

Die Strömungsverhältnisse kehren sich im Schritt b), gemessen an jenen zur Fig. 2a beschriebenen, im Wesentlichen um.

Dabei kann die Blutpumpe 11 stehen. Ist sie, wie in den Figuren optional gezeigt, eine Okklusionspumpe, so verhindert sie in ihrem Ruhezustand einen Rückfluss von Blut in die arterielle Blutleitung 8. Dabei erhöht sie, da stehend bzw. in Ruhe, den Druck stromab der Blutpumpe 11 nicht.

Anstelle oder ergänzend zu einer möglichen Okklusionswirkung der Blutpumpe 11 kann in der arteriellen Blutleitung 8 eine in den Fig. 2a und 2b nicht gezeigte arterielle Schlauchklemme vorgesehen sein. Sie kann in der in Fig. 2b dargestellten Phase ein Strömen von Blut entlang der arteriellen Blutleitung 8 in Richtung weg vom Blutfilter 19 und hin zum Patienten verhindern. Die arterielle Schlauchklemme kann jene mit Bezugzeichen 6 der Fig. 1 sein. Sie kann hiervon abweichend eine andere Schlauchklemme und/oder an anderer Stelle in der arteriellen Blutleitung 8 angeordnet sein.

Die Pumpwirkung, welche zum in Fig. 2b angedeuteten Übertritt von Fluid über die semi-permeable Membran 19c hinweg führt, kann von der in Fig. 2b nicht gezeigten Ultrafiltrationspumpe P03 oder jeder anderen Pumpe auf der Dialysier- oder Hydraulikseite bewirkt sein, etwa von einer Dialysierflüssigkeitspumpe.

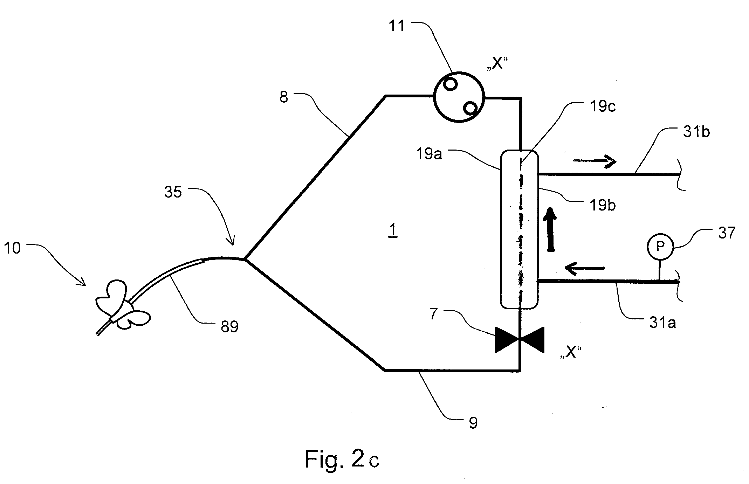

**Fig. 2c** zeigt in der Darstellung der Fig. 2a und der Fig. 2b exemplarisch den Blutschlauchsatz 1 während der optionalen, zwischen der ersten und der zweiten Phase vorgesehenen dritten Phase des Verfahrens.

In der dritten Phase oder Interimsphase (oben als Schritt c) bezeichnet) wird kein oder im Wesentlichen kein Flüssigkeitsvolumen über die semi-permeable Membran 19c verschoben. Vielmehr wird in Schritt c) Flüssigkeit, zumeist frische Dialysierflüssigkeit oder Substituatlösung, etwa aus einem Beutel oder von der Blutbehandlungsvorrichtung hergestellt, entlang der semi-permeablen Membran 19c durch die Dialysierflüssigkeitskammer 19b geführt.

Dabei kann die Blutpumpe 11 stehen. Ist sie, wie in den Figuren optional gezeigt, eine Okklusionspumpe, so verhindert sie in ihrem Ruhezustand einen Rückfluss von Blut in die arterielle Blutleitung 8.

Anstelle oder ergänzend zu einer möglichen Okklusionswirkung der Blutpumpe 11 kann in der arteriellen Blutleitung 8 eine in den Fig. 2a, 2b und 2c nicht gezeigte arterielle Schlauchklemme vorgesehen sein. Sie kann in der in Fig. 2c dargestellten Phase ein Strömen von Blut entlang der arteriellen Blutleitung 8 in Richtung weg vom Blutfilter 19 und hin zum Patienten verhindern. Die arterielle Schlauchklemme kann jene mit Bezugzeichen 6 der Fig. 1 sein. Sie kann hiervon abweichend eine andere Schlauchklemme und/oder an anderer Stelle in der arteriellen Blutleitung 8 angeordnet sein.

Die sich ergebenden Flüssigkeitsströme sind entlang der Membran 19c mittels Pfeilen angedeutet. Sie sind ferner mittels Pfeilen an den Leitungen 31a und 31b angedeutet.

Wie vorstehend ausgeführt fördert die Blutpumpe 11 in dieser dritten Phase (Interimsphase) nicht, so dass es zu keinen Flüssen in der Zugangseinrichtung 89 und in der arteriellen Blutleitung 8 in Richtung zum Blutfilter 19 kommt. Die venöse Schlauchklemme 7 ist dabei geschlossen, was wiederum mit einem "X" neben der Schlauchklemme 7 angedeutet ist. In der venösen Blutleitung 9, die sich dem Blutfilter 19 stromab anschließt, fließt aufgrund der geschlossenen Schlauchklemme 7 ebenfalls kein Blut.

Korpuskulare Bestandteile, die die semi-permeable Membran 19c nicht überwinden können, verbleiben in dieser Interimsphase zwar auf der Blutseite des Blutfilters 19, also auf Seite der Blutkammer 19a, eine Diffusion von membrangängigen Stoffen erfolgt aufgrund des - durch die o. g. Hämokonzentration weiter erhöhten Konzentration an Urämietoxinen - osmotischen Drucks zwischen Blutkammer 19a und Dialysierflüssigkeitskammer 19b hingegen sehr wohl.

Der in der in Fig. 2c gezeigten Phase stattfindende Übertritt allein mittels Diffusion über die semi-permeable Membran 19c, also von der Blutkammer 19a in die Dialysierflüssigkeitskammer 19b kann als "Dialyse" oder "Hämodialyse" bezeichnet werden.

Die Pumpwirkung, welche zum in Fig. 2c angedeuteten Strom von Fluid entlang, aber nicht über die semi-permeable Membran 19c hinweg, führt, kann von der in Fig. 2c nicht gezeigten Ultrafiltrationspumpe P03 oder jeder anderen Pumpe auf der Dialysier- oder Hydraulikseite (wie etwa einer Zweikammer-Dosierpumpe, deren Kammern wie vorstehend alternierend das Kammervolumen entsprechend der o. g. Phasen zur Filtration oder Substitution bereithalten) bewirkt sein, etwa von einer Dialysierflüssigkeitspumpe.

Nicht mittels Figuren gezeigt ist eine Kombination der o. g. Schritte a) und c) bzw. b) und c). Für solche Kombinationen können die in den Fig. 2a und 2c bzw. 2b und 2c mittels Pfeilen dargestellten Ströme jeweils gemeinsam vorliegen.

Die folgenden Ausführungen erläutern konkretere, exemplarische Ausgestaltungen oder Ausführungsformen des Verfahrens.

Das Blutvolumen im Blutfilter 19 lässt sich nach Angaben der Erfinder um bis zu 33% (f=0,33) aufkonzentrieren. Bei einem Blutfiltervolumen $V_{DB}$ von beispielsweise 120ml errechnet sich das maximal mögliche Schlagvolumen $V_s$ somit zu:

(1)

$$V_S = V_{DB} \cdot \frac{f}{1-f} = 120ml \cdot \frac{0{,}33}{1-0{,}33} = 60ml$$

Nachweis der Gleichung 1 (in Analogie zur geometrischen Reihe):
Ein aus der Blutkammer 19a verdrängtes Volumen f* $V_{DB}$ wird wieder, vorzugsweise mittels frischer Substituatlösung, in den Blutkreislauf 1 rückgeführt. Anschließend wird erneut ein Volumen f* f* $V_{DB}$ verdrängt und optional verworfen, welches anschließend wieder durch frische Flüssigkeit (also Flüssigkeit, die noch nicht mit Blut in Kontakt gelangt war) aufgefüllt oder ersetzt wird, und so fort:

(2)

$$V_S = V_{DB} \cdot (f + f^2 + f^3 + \cdots) = f \cdot V_{DB} \cdot \sum_{n=0} f^n$$

Umformungen liefern für den vorstehenden Ausdruck:

(3)

$$\sum_{n=0} f^n = \frac{1}{1-f} \quad , f < 1$$

und für das Schlagvolumen $V_s$:

(4)

$$V_S = f \cdot V_{DB} \cdot \sum_{n=0} f^n = V_{DB} \cdot \frac{f}{1-f}$$

Im Detail sind die beiden folgenden sich wiederholenden Verfahrensschritte 1) und 2) notwendig, um mit der Single-Needle-Therapie zu behandeln:

1) Mittels der Blutpumpe wird bei geschlossener venöser Schlauchklemme 7 (Fig. 2a) Patientenblut in den Blutfilter 19 gefördert, während im vorliegenden Beispiel ca. 33 % des Blutvolumens, durch das Abfiltrieren von Plasmawasser über die semi-permeable Membran 19c hinweg in den hydraulischen Teil, im Blutfilter 19 aufkonzentriert wird.

Das Abfiltrieren erfolgt entweder über aktive hydraulikseitige Ultrafiltration z. B. mittels der Ultrafiltrationspumpe P03 oder ohne Einsatz von Pumpen auf der Hydraulikseite (Dialysierseite) durch Volumenverschiebung während des Abbaus der durch die Blutpumpe 11 erzeugten Erhöhung des Drucks in der Blutkammer 19a des Blutfilters 19.
Das maximal zu verdrängende Volumen $V_S$ ("Schlagvolumen") ist von der Größe des blutseitigen Volumens $V_{DB}$ der Blutkammer 19a des Blutfilters 19 und der Aufkonzentration $f$ abhängig und berechnet sich gemäß:

(5)

$$V_S = V_{DB} \cdot \frac{f}{1-f}$$

Die Hämokonzentration im Blutfilter 19 führt zu einer Abnahme der Permeabilität der semi-permeablen Membran 19c, was zu einem Anstieg des Drucks in der Blutkammer 19a führt. Dieser kann über einen Drucksensor zwischen Blutpumpe 11 und Blutfilter 19, z. B. den Präfilterdrucksensor 33c, gemessen oder anhand der elektrischen Leistungsaufnahme der Blutpumpe 11 abgeschätzt werden. Der gemessene Druck, egal wo gemessen, kann zur Regelung des Verfahrens verwendet werden, die erfindungsgemäßen Vorrichtungen können entsprechend konfiguriert sein. Der Druck kann ergänzend oder alternativ, insbesondere bei geschlossener venöser Schlauchklemme 7, auch am venösen Drucksensor 33b stromabwärts des Blutfilters 19 gemessen werden.
Eine weitere Möglichkeit eines Feedbacks bietet die Analyse der von der peristaltischen Blutpumpe 11 generierten Druckpulse, die z. B. mittels des hydraulikseitigen Drucksensor 37 gemessen werden können. Bei abnehmender Membranpermeabilität wird das empfangene Drucksignal am hydraulischen Drucksensor 37 geringer.

Bei Erreichen eines vorbestimmten Druckwertes in der Blutkammer 19a wird die Blutpumpe 11 gestoppt und die venöse Schlauchklemme 7 geöffnet, während mittels Überdrucks im hydraulischen Teil Volumen über die semi-permeable Membran 19c hinweg in den Blutschlauchsatz 1 zurückgepumpt wird.

Entlang der venösen Blutleitung 9 fließt das zurückgewonnene Volumen in Richtung zum Patienten zurück. Die über die semi-permeable Membran 19c transportierten Volumina werden optional bilanziert. Der Reinigungseffekt wird im Blutfilter 19 einerseits durch diffusive Transportprozesse bei vorhandenem Dialysierflüssigkeits- bzw. Dialysatfluss und durch den konvektiven Transport des zu verdrängenden Plasmawassers bewirkt.

Die auf Wunsch nach Gleichung (1) zu erreichenden Schlagvolumina sind mit typischen Volumina in Standard-Single-Needle-Therapien vergleichbar.

Der in der in Fig. 2b gezeigten Phase stattfindende Übertritt von Fluid über die semi-permeable Membran 19c, also von der Dialysierflüssigkeitskammer 19b in die Blutkammer 19a kann als "negative Ultrafiltration" bezeichnet werden. Sie kann zum Kompensieren der in Fig. 2a gezeigten Phase in manchen Ausführungsformen zumindest phasenweise akzeptierten Fehlbilanz z. B. mittels Substituatgabe auf der Hydraulikseite unterstützt werden. Die Fehlbilanz, die sich z. B. dadurch ergeben haben kann, dass Fluid aus der Hydraulikseite oder Dialysierseite (also rechts der semi-permeablen Membran 19c in Fig. 2a) verworfen wurde, kann hierdurch korrigiert oder kompensiert werden.

Die Fehlbilanz, die in der in Fig. 2a gezeigten Phase entsteht, und welche in der in Fig. 2b gezeigten Phase kompensiert wird, kann gezielt gewollt und in ihrer Größe optional vorbestimmt sein.

**Bezugszeichenliste**

| | |
|---|---|
| 1 | extrakorporaler Blutkreislauf, Blutschlauchsatz |
| 2 | Blutkassette |
| 3 | erste Fluidführung des automatischen Substituatkonnektors 18b |
| 4 | Blutbehandlungsvorrichtung |
| 5 | zweite Fluidführung des automatischen Substituatkonnektors 18b |
| 6 | arterielle Schlauchklemme |
| 7 | venöse Schlauchklemme |
| 8 | arterielle Blutleitung |
| 9 | venöse Blutleitung |
| 10 | Zugangseinrichtung |
| 11 | Blutpumpe |
| 13 | Zugabestelle für Substituatflüssigkeit (Prädilution) |
| 14 | Zugabestelle für Substituatflüssigkeit (Postdilution) |
| 15 | arterieller Luft-Blut-Detektor |
| 17 | Substituatpumpe |
| 18a | automatischer Substituatport |
| 18b | automatischer Substituatkonnektor |
| 19 | Blutfilter |
| 19a | Blutkammer |
| 19b | Dialysierflüssigkeitskammer |
| 19c | semi-permeable Membran |
| 21 | venöse Luftabscheidekammer |
| 23 | Rückschlagventil des Substituatkanals |
| 25 | venöser Luft-Blut-Detektor oder Luft-Substituatflüssigkeit-Blut-Detektor |
| 26 | Druckluftquelle |
| 28 | Steuervorrichtung, Steuer- und/oder Regelvorrichtung |
| 31a | Dialysierflüssigkeitszulaufleitung |
| 31b | Dialysatablaufleitung |
| 33a, b | Drucksensoren |
| 33c | Präfilterdrucksensor |
| 35 | Y-förmiger-Verbinder |
| 37 | Drucksensor |
| 40 | Hydraulik-Bilanzkammer |
| 45 | Drain-Leitung |
| 89 | Patientenschlauchleitung |

V19    Ventil der Dialysatablaufleitung 31b
V22    Ventil
V24    Ventil der Dialysierflüssigkeitszulaufleitung 31a
V25    Ventil der Dialysatablaufleitung 31b
V26    Ventil
V28    Ventil der Dialysatablaufleitung 31b
V31    Ventil der ersten Fluidführung 3 des automatischen Substituatkonnektors 18b
V32    Ventil der zweiten Fluidführung 5 des automatischen Substituatkonnektors 18b
V33    erstes Ventil der Drain-Leitung 45
P03    Ultrafiltrationspumpe

**Patentansprüche**

1. Steuervorrichtung (28) zum Steuern oder Regeln einer Blutbehandlungsvorrichtung (4) zum Behandeln von Blut mittels eines Single-Needle-Verfahrens, wobei die zu steuernde oder zu regelnde Blutbehandlungsvorrichtung (4) aufweist:

   - eine Blutpumpe (11), verbindbar mit einem Blutschlauchsatz (1) zum Fördern von Blut durch den Blutschlauchsatz (1), wobei die Blutpumpe (11) in einer Entnahmerichtung betreibbar ist;
   - eine Rückgabepumpe, angeordnet, um im Gebrauch der Blutbehandlungsvorrichtung (4) Fluid in Richtung zum Blutschlauchsatz (1) oder einem Abschnitt hiervon und/oder zum Patienten in einer Rückgaberichtung zu fördern;
   wobei die Steuervorrichtung (28) konfiguriert ist, mittels der Blutbehandlungsvorrichtung (4) ein Verfahren auszuführen, welches folgende Schritte umfasst:

   a) Betreiben der Blutpumpe (11) in einer Entnahmerichtung, in welcher Blut aus dem Patientenblutkreislauf entnommen und entlang einer Patientenschlauchleitung (89) in den Blutschlauchsatz (1), in welchen ein Blutfilter (19) mit einer Blutkammer (19a) und einer Dialysierflüssigkeitskammer (19b) eingeschaltet ist, wobei Blutkammer (19a) und Dialysierflüssigkeitskammer (19b) durch eine semi-permeable Membran (19c) voneinander getrennt sind, gepumpt wird; und
   b) Betreiben der Rückgabepumpe derart, dass Blut aus dem Blutschlauchsatz (1) heraus und entlang der Patientenschlauchleitung (89) in den Patientenblutkreislauf zurück gepumpt wird;

   wobei die Schritte a) und b) mehrfach alternierend durchgeführt werden;
   wobei das Verfahren zudem den folgenden Schritt umfasst

   - Steuern und/oder Überwachen des Verfahrens durch oder unter Analyse von Druckmesswerten und/oder der Motorstromaufnahme mindestens einer Pumpe;

   **gekennzeichnet dadurch, dass** Blut zwischen seiner Entnahme in Schritt a) und seiner Rückgabe in Schritt b) nicht in einer blutseitigen Single-Needle-Kammer gespeichert wird.

2. Steuervorrichtung (28) nach Anspruch 1, wobei das Verfahren weiter umfasst:

   - Schließen einer venösen Schlauchklemme (7), während oder bevor Blut in Schritt a) vom Patienten in Richtung zum Blutfilter (19) gefördert wird;
   - Bewirken des Übertritts von Plasmawasser des geförderten Bluts über die semi-permeable Membran (19c) des Blutfilters (19) durch Aufbauen eines Druckunterschieds zwischen der Dialysierflüssigkeitskammer (19b) und der Blutkammer (19a);
   - Öffnen der venösen Schlauchklemme (7);
   - Bewirken des Übertritts von Flüssigkeit von der Dialysierflüssigkeitskammer (19b) über die semi-permeable Membran (19c) in die Blutkammer (19a) des Blutfilters (19) durch Aufbauen eines geeigneten Drucks auf der Hydraulikseite, während in Schritt b) entlang der Patientenschlauchleitung (89) Blut in den Patientenblutkreislauf zurück gepumpt wird.

3. Steuervorrichtung (28) nach Anspruch 1 oder 2, wobei der Blutschlauchsatz (1) aufweist:

   - eine Patientenschlauchleitung (89) zu sowohl der Entnahme von Blut aus dem Patientenblutkreislauf in den Blutschlauchsatz (1) als auch dessen Rückgabe aus dem Blutschlauchsatz (1) in den Patientenblutkreislauf;
- einen Y-förmigen Verbinder (35) oder Drei-Wege-Verbinder, welcher verbunden ist mit der Patientenschlauchleitung (89);

wobei der Blutschlauchsatz (1) keine blutseitige Single-Needle-Kammer aufweist oder mit keiner blutseitigen Single-Needle-Kammer verbunden ist.

4. Blutbehandlungsvorrichtung (4) zum Durchführen einer Single-Needle-Blutbehandlung, wobei die Blutbehandlungsvorrichtung (4) aufweist:

- eine Blutpumpe (11), verbindbar mit einem Blutschlauchsatz (1) zum Fördern von Blut durch den Blutschlauchsatz (1), wobei die Blutpumpe (11) in einer Entnahmerichtung betreibbar ist;
- eine Rückgabepumpe, angeordnet, um im Gebrauch der Blutbehandlungsvorrichtung (4) Fluid in Richtung zum Blutschlauchsatz (1) oder einem Abschnitt hiervon und/oder zum Patienten in einer Rückgaberichtung zu fördern;
- eine Steuervorrichtung (28), konfiguriert zum Steuern oder Regeln der Blutbehandlungsvorrichtung (4);

wobei die Steuervorrichtung (28) programmiert ist zum Durchführen einer Single-Needle-Blutbehandlung mittels eines Verfahrens, welches folgende Schritte umfasst:

a) Betreiben der Blutpumpe (11) in einer Entnahmerichtung, in welcher Blut aus dem Patientenblutkreislauf entnommen und in den Blutschlauchsatz (1), in welchen ein Blutfilter (19) mit einer Blutkammer (19a) und einer Dialysierflüssigkeitskammer (19b) eingeschaltet ist, wobei Blutkammer (19a) und Dialysierflüssigkeitskammer (19b) durch eine semi-permeable Membran (19c) voneinander getrennt sind, gepumpt wird; und
b) Betreiben der Rückgabepumpe derart, dass Blut aus dem Blutschlauchsatz (1) heraus und in den Patientenblutkreislauf zurück gepumpt wird;

wobei die Schritte a) und b) mehrfach alternierend durchgeführt werden;
wobei das Verfahren zudem den folgenden Schritt umfasst
Steuern und/oder Überwachen des Verfahrens durch oder unter Analyse von Druckmesswerten und/oder der Motorstromaufnahme mindestens einer Pumpe;
**gekennzeichnet dadurch, dass** Blut zwischen seiner Entnahme in Schritt a) und seiner Rückgabe in Schritt b) nicht in einer blutseitigen Single-Needle-Kammer zwischengespeichert wird.

5. Blutbehandlungsvorrichtung (4) nach Anspruch 4, ausgestaltet als Hämodialysevorrichtung, Hämofiltrationsvorrichtung oder als Hämodiafiltrationsvorrichtung, insbesondere als eine Vorrichtung für die chronische Nierenersatztherapie oder für die kontinuierliche Nierenersatztherapie (CRRT = *continuous renal replacement therapy*).

6. Blutbehandlungsvorrichtung (4) nach einem der Ansprüche 4 oder 5, wobei der Blutschlauchsatz (1) aufweist:

- eine Patientenschlauchleitung (89) zu sowohl der Entnahme von Blut aus dem Patientenblutkreislauf in den Blutschlauchsatz (1) als auch dessen Rückgabe aus dem Blutschlauchsatz (1) in den Patientenblutkreislauf;
- einen Y-förmigen Verbinder (35) oder Drei-Wege-Verbinder, welcher verbunden ist mit der Patientenschlauchleitung (89);

wobei der Blutschlauchsatz (1) keine blutseitige Single-Needle-Kammer aufweist oder mit keiner blutseitigen Single-Needle-Kammer verbunden ist.

**Claims**

1. A control device (28) for controlling or regulating a blood treatment apparatus (4) for treating blood by means of a single-needle method, wherein the blood treatment apparatus (4) to be controlled or regulated comprises:

- a blood pump (11), connectable to a blood hose set (1) for conveying blood through the blood hose set (1), wherein the blood pump (11) is operable in a withdrawal direction;
- a return pump, arranged to convey fluid towards the blood hose set (1) or a portion thereof and/or towards the patient in a return direction during use of the blood treatment device (4);

wherein the control device (28) is configured, to perform a method by means of the blood treatment apparatus (4), the method encompassing the following steps:

a) operating the blood pump (11) in a withdrawal direction, in which blood is withdrawn from the patient blood circuit and pumped along a patient hose line (89) into the blood hose set (1), into which a blood filter (19) with a blood chamber (19a) and a dialysis liquid chamber (19b) is integrated, the blood chamber (19a) and the dialysis liquid chamber (19b) being separated from one another by a semi-permeable membrane (19c); and

b) operating the return pump such that blood is pumped out of the blood hose set (1) and pumped along the patient hose line (89) back into the patient blood circuit;

wherein steps a) and b) are carried out alternately several times;

wherein the method further comprises the following step:

- controlling and/or monitoring the method by or under analysis of the pressure measurement values and/or the motor electricity consumption of at least one pump;

**characterized in that**, that blood is not stored in a blood-side single-needle chamber between its withdrawal in step a) and its return in step b).

2.  The control device (28) according to claim 1, wherein the method further comprises:

- closing a venous hose clamp (7) during or before blood is conveyed from the patient towards the blood filter (19) in step a);

- effecting the passage of plasma water of the conveyed blood via the semi-permeable membrane (19c) of the blood filter (19) by establishing a pressure difference between the dialysis liquid chamber (19b) and the blood chamber (19a);

- opening the venous hose clamp (7);

- effecting the passage of liquid from the dialysis liquid chamber (19b) via the semi-permeable membrane (19c) into the blood chamber (19a) of the blood filter (19) by establishing a suitable pressure on the hydraulic side, while in step b) blood is being pumped back into the patient blood circuit along the patient hose line (89).

3.  The control device (28) according to claim 1 or 2, wherein the blood hose set (1) comprises:

- a patient hose line (89) for both the withdrawal of blood from the patient blood circuit into the blood hose set (1) and its return from the blood hose set (1) into the patient blood circuit;

- a Y-shaped connector (35) or three-way connector connected to the patient hose line (89);

wherein the blood hose set (1) does not have a blood-side single-needle chamber or is not connected to a blood-side single-needle chamber.

4.  A blood treatment apparatus (4) for performing a single-needle blood treatment, wherein the blood treatment apparatus (4) comprises:

- a blood pump (11), connectable to a blood hose set (1) for conveying blood through the blood hose set (1), wherein the blood pump (11) is operable in a withdrawal direction;

- a return pump, arranged to convey fluid towards the blood hose set (1) or a portion thereof and/or towards the patient in a return direction during use of the blood treatment device (4);

- a control device (28), configured for controlling or regulating the blood treatment apparatus (4);

wherein the control device (28) is programmed to perform a single-needle treatment by means of a method comprising the following steps:

a) operating the blood pump (11) in a withdrawal direction, in which blood is withdrawn from the patient blood circuit and pumped into the blood hose set (1), into which a blood filter (19) with a blood chamber (19a) and a dialysis liquid chamber (19b) is integrated, the blood chamber (19a) and the dialysis liquid chamber (19b) being separated from one another by a semi-permeable membrane (19c); and

b) operating the return pump such that blood is pumped out of the blood hose set (1) and pumped back into the patient blood circuit;

wherein the method further comprises the following step:

- controlling and/or monitoring the method by or under analysis of the pressure measurement values and/or the motor electricity consumption of at least one pump;

**characterized in that**, that blood is not stored in a blood-side single-needle chamber between its withdrawal in step a) and its return in step b).

5.  A blood treatment apparatus (4) according to claim 4, embodied as a hemodialysis apparatus, hemofiltration apparatus or as a hemodiafiltration apparatus, in particular as an apparatus for chronic renal replacement therapy or for continuous renal replacement therapy (CRRT = *continuous renal replacement therapy*).

6.  The blood treatment apparatus (4) according to anyone of the claims 4 or 5, wherein the blood hose set (1) comprises:

- a patient hose line (89) for both the withdrawal of blood from the patient blood circuit into the blood hose set (1) and its return from the blood hose set (1) into the patient blood circuit;
- a Y-shaped connector (35) or three-way connector connected to the patient hose line (89);

wherein the blood hose set (1) does not have a blood-side single-needle chamber or is not connected to a blood-side single-needle chamber.

## Revendications

1.  Un dispositif de commande (28) pour commander ou réguler un appareil de traitement du sang (4) destiné à traiter le sang au moyen d'un procédé single-needle, où l'appareil de traitement du sang (4) à commander ou réguler comprend :

- une pompe à sang (11), raccordable à un set de tuyaux sanguins (1) pour acheminer du sang au travers du set de tuyaux sanguins (1), où la pompe à sang (11) peut être actionnée dans une direction de prélèvement;
- une pompe de retour, agencée pour acheminer du fluide vers le set de tuyaux sanguins (1) ou une partie de celui-ci et/ou vers le patient dans une direction de retour lors de l'utilisation de l'appareil de traitement du sang (4); où le dispositif de commande (28) est configuré pour exécuter un procédé au moyen de l'appareil de traitement du sang (4), le procédé comprenant les étapes suivantes :

a) actionner la pompe à sang (11) dans une direction de prélèvement dans laquelle le sang est prélevé du circuit sanguin du patient et pompé le long d'un conduit de tuyau du patient (89) dans le set de tuyaux sanguins (1), dans lequel est intégré un filtre sanguin (19) doté d'une chambre à sang (19a) ainsi que d'une chambre à liquide de dialyse (19b), la chambre à sang (19a) ainsi que la chambre à liquide de dialyse (19b) étant séparées l'une de l'autre par une membrane semi-perméable (19c); et
b) actionner la pompe de retour de manière à ce que le sang soit pompé hors du set de tuyaux sanguins (1) et renvoyé le long du conduit de tuyau du patient (89) dans le circuit sanguin du patient;

où les étapes a) et b) sont effectuées plusieurs fois en alternance;
où le procédé comprend en outre l'étape suivante :

- commander et/ou surveiller le procédé par ou sous analyse des valeurs de mesure de la pression et/ou de la consommation d'électricité du moteur d'au moins une pompe;

**caractérisé en ce que**, le sang ne soit pas stocké dans une chambre single-needle côté sang entre son prélèvement à l'étape a) et son retour à l'étape b).

2.  Le dispositif de commande (28) selon la première revendication, où le procédé consiste en outre à :

- fermer une pince de tuyau veineux (7) pendant ou avant que le sang ne soit acheminé du patient vers le filtre sanguin (19) à l'étape a);
- effectuer le passage de l'eau plasmatique du sang acheminé via la membrane semi-perméable (19c) du filtre sanguin (19) en créant une différence de pression entre la chambre de liquide de dialyse (19b) et la chambre à sang (19a);
- ouvrir la pince de tuyau veineux (7);
- effectuer le passage de liquide depuis la chambre à liquide de dialyse (19b) dans la chambre à sang (19a) du filtre sanguin (19) via la membrane semi-perméable (19c) en créant une pression appropriée côté hydraulique, pendant qu'à l'étape b) le sang est pompé en retour dans le circuit sanguin du patient le long du conduit de tuyau du patient (89).

3. Le dispositif de commande (28) selon la revendication 1 ou 2, où le set de tuyaux sanguins (1) comprend :

- un conduit de tuyau de patient (89) pour à la fois le prélèvement de sang depuis le circuit sanguin du patient dans le set de tuyaux sanguins (1) et son retour depuis le set de tuyaux sanguins (1) dans le circuit sanguin du patient;
- un connecteur en forme de Y (35) ou un connecteur à trois voies relié au conduit de tuyau de patient (89);

où le set de tuyaux sanguins (1) ne comporte pas de chambre single-needle côté sang ou n'est pas relié à une chambre single-needle côté sang.

4. Un appareil de traitement du sang (4) pour exécuter un traitement du sang à single-needle, où l'appareil de traitement du sang (4) comprend :

- une pompe à sang (11), raccordable à un set de tuyaux sanguins (1) pour acheminer du sang au travers du set de tuyaux sanguins (1), où la pompe à sang (11) peut être actionnée dans une direction de prélèvement;
- une pompe de retour, agencée pour acheminer du fluide vers le set de tuyaux sanguins (1) ou une partie de celui-ci et/ou vers le patient dans une direction de retour lors de l'utilisation de l'appareil de traitement du sang (4);
- un dispositif de commande (28), configuré pour commander ou réguler l'appareil de traitement du sang (4);

où le dispositif de commande (28) est programmé pour exécuter un traitement du sang à single-needle au moyen d'un procédé qui comprend les étapes suivantes consistant à :

a) actionner la pompe à sang (11) dans une direction de prélèvement dans laquelle le sang est prélevé du circuit sanguin du patient et pompé dans le set de tuyaux sanguins (1), dans lequel est activé un filtre sanguin (19) doté d'une chambre à sang (19a) ainsi que d'une chambre à liquide de dialyse (19b), la chambre à sang (19a) ainsi que la chambre à liquide de dialyse (19b) étant séparées l'une de l'autre par une membrane semi-perméable (19c); et
b) actionner la pompe de retour de manière à ce que le sang soit pompé hors du set de tuyaux sanguins (1) et renvoyé dans le circuit sanguin du patient;

où les étapes a) et b) sont effectuées plusieurs fois en alternance;

où le procédé comprend en outre l'étape suivante consistant à :

- commander et/ou surveiller le procédé par ou sous analyse des valeurs de mesure de la pression et/ou de la consommation d'électricité du moteur d'au moins une pompe;

**caractérisé en ce que**, le sang ne soit pas stocké dans une chambre single-needle côté sang entre son prélèvement à l'étape a) et son retour à l'étape b).

5. Un appareil de traitement du sang (4) selon la revendication 4, conçu comme un appareil d'hémodialyse, un appareil d'hémofiltration ou comme un appareil d'hémodiafiltration, notamment comme un appareil pour une thérapie de remplacement rénal chronique ou pour une thérapie de remplacement rénal continu (CRRT = *continuous renal replacement therapy*).

6. L'appareil de traitement du sang (4) selon l'une quelconque des revendications 4 ou 5, où le set de tuyaux sanguins (1) comprend :

- un conduit de tuyau de patient (89) pour à la fois le prélèvement de sang depuis le circuit sanguin du patient dans le set de tuyaux sanguins (1) et son retour depuis le set de tuyaux sanguins (1) dans le circuit sanguin du patient;
- un connecteur en forme de Y (35) ou un connecteur à trois voies relié au conduit de tuyau de patient (89);

où le set de tuyaux sanguins (1) ne comporte pas de chambre single-needle côté sang ou n'est pas relié à une chambre single-needle côté sang.

Fig. 1

Fig. 2a

Fig. 2b

EP 3 773 791 B1

Fig. 2c

EP 3 773 791 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0143064 A1

- WO 2010121819 A1

**In der Beschreibung aufgeführte Nicht-Patentliteratur**

- **DRUKKER** ; **PARSONS** ; **MAHER**. Single-Needle. Kluwer Academic Publishers, 2004, 365